(19) Europäisches Patentamt
European Patent Office
Office européen des brevets

(11) **EP 1 380 333 B1**

(12) **EUROPEAN PATENT SPECIFICATION**

(45) Date of publication and mention
of the grant of the patent:
**05.12.2007 Bulletin 2007/49**

(51) Int Cl.:
*B01D 39/20* (2006.01)        *A61F 5/441* (2006.01)

(21) Application number: **03254336.5**

(22) Date of filing: **08.07.2003**

(54) **Odour absorbing filters**

Geruchabsorbierende Filter

Filtre absorbant les odeurs

(84) Designated Contracting States:
**AT BE BG CH CY CZ DE DK EE ES FI FR GB GR HU IE IT LI LU MC NL PT RO SE SI SK TR**

(30) Priority: **09.07.2002 GB 0215828**

(43) Date of publication of application:
**14.01.2004 Bulletin 2004/03**

(73) Proprietor: **WELLAND MEDICAL LIMITED Crawley,**
**West Sussex RH10 2TU (GB)**

(72) Inventors:
• **Smith, Rory James Maxwell**
**North Yorkshire BD23 5EB (GB)**
• **Bird, Paul**
**West Sussex RH10 3RQ (GB)**

(74) Representative: **Griffin, Kenneth David**
**Saunders & Dolleymore,**
**9, Rickmansworth Road**
**Watford,**
**Hertfordshire WD18 0JU (GB)**

(56) References cited:
**EP-A- 0 191 646**        **WO-A-98/26808**
**GB-A- 1 235 874**        **US-A- 5 281 437**

• **PATENT ABSTRACTS OF JAPAN vol. 2000, no. 21, 3 August 2001 (2001-08-03) & JP 2001 104370 A (OJI PAPER CO), 17 April 2001 (2001-04-17)**
• **PATENT ABSTRACTS OF JAPAN vol. 12, no. 382, 12 October 1988 (1988-10-12) & JP 63 130138 A (HOKUETSU TANSO KOGYO KK), 2 June 1988 (1988-06-02)**
• **PATENT ABSTRACTS OF JAPAN vol. 2000, no. 24, 11 May 2001 (2001-05-11) & JP 2001 179016 A (KANAI HIROAKI), 3 July 2001 (2001-07-03)**
• **PATENT ABSTRACTS OF JAPAN vol. 1995, no. 04, 31 May 1995 (1995-05-31) & JP 07 024256 A (NIPPON SOKEN INC), 27 January 1995 (1995-01-27)**

**Description**

The present invention relates to odour adsorbing or deodorizing filters, in particular to odour adsorbing filters suitable for medical use, for example for incorporation in an ostomy collection bag or in a wound dressing, in order to adsorb the unpleasant odours emanating from an ostomy bag or an infected a wound, thereby preventing transmission of such odours. Such an odour adsorbing filter is disclosed in EP-A-0191646.

Ostomy collection bags are discreet pouches or bags which are used to collect the liquid, semi-solid or solid output from a stoma, which is an artificial opening in the abdomen of a patient who has undergone colostomy or ileostomy surgery. A stoma is created in the abdomen wall when a patient has suffered from a bowel obstruction, inflammatory bowel disease, faecal incontinence, trauma, cancer or the like and it enables the bowels to empty.

Ostomy bags can be closed bags that have to be changed 2-3 times a day or alternatively they may be drainable bags that are drained frequently and are then changed every 2-3 days. They contain a filter that allows flatus to be released through a charcoal layer that helps to remove faecal odour.

Since the composition of faeces varies with diet the chemistry of the odour emanating from faeces is chemically complex. One cause of faecal odour is due to the presence of a family of sulphur based chemicals known as mercaptans. Other major chemical groups that contribute to the odour are indoles and skatoles, both of which are large cyclic organic compounds.

Filters suitable for use in ostomy bags have been made from microporous activated carbon. The British Standard Test for filter performance challenges the filter with hydrogen sulphide gas. In order to pass and exceed the requirements of the test chemical treatment of the carbon with, for example, copper, chromium, manganese or iron salts has been necessary. The salt is impregnated and deposited in the microporous activated carbon. Whilst the salt deposited on the surface of the carbon interacts chemically with the hydrogen sulphide and thereby removes it, the impregnating and deposited salt blocks the surface of the carbon with the result that the pores in the body of the structure are much less available to other chemical species for adsorption. Consequently, other odour causing chemicals are not adsorbed so efficiently.

It has now been found that by selecting a specific mixture of a chemically modified particulate activated carbon and an unmodified particulate activated carbon and impregnating the mixture into a matrix or substrate a filter can be constructed that is very effective in removing the different groups of odour-producing chemicals when the filter is incorporated in an ostomy bag. Alternatively, such a filter can be incorporated into a dressing for application to odour-producing wounds, or into fluid collection devices used to collect infected bodily fluids, such as wound exudates. The filter according to the invention may also be used in respirators, filters used in air conditioning units, as well as for deodorising sewage plants.

According to the present invention there is provided an odour adsorbing filter suitable for use in medical appliances which comprises a filtration layer formed of a matrix or substrate impregnated with activated carbon together with a binder and sandwiched between cover layers characterised in that the matrix or substrate is impregnated with a mixture comprising from 50 to 95 % w/w particulate chemically modified activated carbon and from 5 to 50 % w/w particulate activated carbon so that the ratio of chemically modified activated carbon to activated carbon is from 50:50 to 95:5.

According to another aspect of the invention there is provided an ostomy bag incorporating an odour adsorbing filter as defined above.

According to yet another aspect of the present invention there is provided a dressing for a wound said dressing incorporating an odour adsorbing filter as defined above.

Preferably the matrix or substrate is impregnated with a mixture comprising from 70 to 87 % w/w particulate chemically modified activated carbon and from 13 to 30 % w/w particulate activated carbon so that the ratio of chemically modified activated carbon to activated carbon is from 70:30 to 87:13. The mixture may comprise from 70 to 80% w/w particulate chemically modified activated carbon and from 20 to 30% w/w particulate activated carbon so that the ratio of chemically modified activated carbon to activated carbon is from 70:30 to 80:20.

The matrix or substrate is most preferably impregnated with a mixture comprising of 75% w/w particulate chemically modified activated carbon and 25% w/w particulate activated carbon.

Alternatively the matrix may be impregnated with a mixture of particulate chemically modified activated carbon and particulate activated carbon where the ratio is from 55:45 to 90:10, preferably from 60:40 to 85:15, most preferably 65:35.

The matrix or substrate that is impregnated with the mixture of activated carbons according to the invention may be a fabric, such as a non-woven fabric, e.g. felt, or a knitted fabric. Alternatively, the matrix may be a foam, for example a polyurethane foam or any other open cell or reticulated foam, or paper. When the deodorizing filter is to be used in a wound dressing the matrix may be a textile fabric or a foam.

The chemically modified particulate activated carbon is prepared by impregnating a starting material, which may be wood, coconut shell or fabric, with a salt of copper, chromium, manganese or iron, or organic material such as tetra ethylenediamine before being carbonised in a furnace. The preferred salt is copper nitrate. The starting material is impregnated with from 9 to 15% *(w/w)*, preferably from 11 to 13% *(w/w)*, most preferably 12% *(w/w)* measured as copper oxide using atomic absorption spectroscopy.

Following carbonisation the treated carbon is activated using steam at high pressure. Activation usually takes place at 800 to 1000°C under strictly controlled atmospheric conditions and in the presence of $CO_2$ and water vapour.

Following activation the carbon is ground, for instance using a ball mill, to reduce the particle size such that 0% remains on a sieve of 40 micron mesh size and 80% will pass through a sieve having a mesh size of 32.5 micron.

The untreated particulate activated carbon component of the filtration layer is prepared by the same method but without the impregnation step.

In order to make the filter of the invention, the selected mixture of carbons i.e. particulate chemically modified activated carbon and the particulate activated carbon are weighed into a rotary mixing machine, which is allowed to operate until complete and intimate mixing is achieved. This usually takes in the order of 30 minutes.

The desired matrix or substrate, which may be a felt type non-woven fabric, is passed over a conveyor. The intimate mixture of particulate carbons is applied from above and drawn into the body of the matrix by the application of a vacuum to the underside of the conveyor, using the process as described in US patent No. 5 281 437.

In order to stabilise the composite structure of matrix impregnated with the activated carbon mixture the retained particulate carbon is fixed with a binder. The binder may be applied to the impregnated matrix by means of an applicator roller that receives the binder solution or suspension from a reservoir via a spreader that spreads binder over the surface of the applicator roller. From 10% to 25% w/w of binder may be applied to the impregnated matrix, preferably from 15% to 19% w/w.

Binders for use in the present invention may be selected from natural or synthetic latexes, for example styrene butadiene, acrylonitrile butadiene, acrylic methyl methacrylate polyvinyl alcohol, polyvinyl acetate, melamineformaldehyde resins or they may comprise solutions of starch, carboxymethyl cellulose, methyl cellulose or sodium silicate. The preferred binder is an acrylonitrile binder.

Following the application of the binder a net-like thermofusible layer, which may be made from polyurethane, polyethylene, ethyl vinylacetate, nylon or similar low melting point materials, is applied to each surface of the impregnated matrix using a belt laminator to form a composite structure.

A film of relatively fluid impervious material is then laminated to each surface of the composite structure to provide fluid impervious cover layers. The film of fluid impervious material comprises ethyl vinylacetate, polyvinylchloride, polyurethane, polyethylene, etc. The preferred film consists of a co-extruded film of ethyl vinyl acetate, polyvinyl dichloride and ethyl vinyl acetate known as Cryovac (Registered Trade Mark).

The resulting multi-layer composite is cut into discs, preferably about 25 mm in diameter, using a rotary or flat bed press.

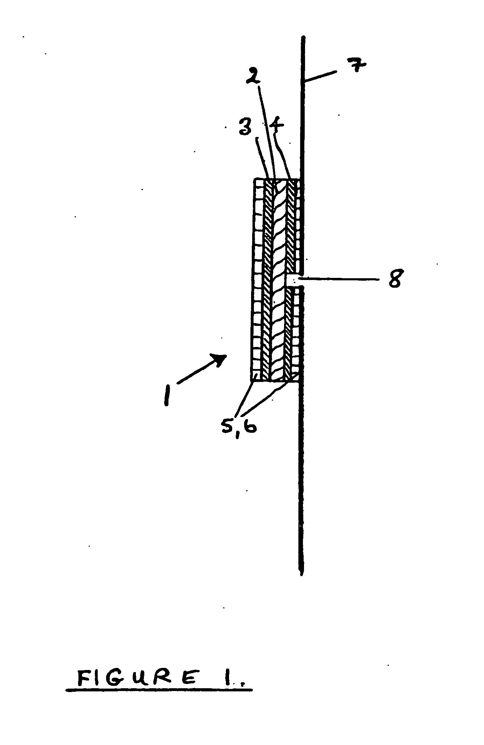

An odour adsorbing filter in accordance with the invention will now be described by way of reference to the accompanying drawing (Figure 1) which is a section through a filter attached to the wall of an ostomy bag.

The filter 1 consists of a filtration layer 2 composed of a felt matrix impregnated with a mixture of 75% particulate chemically modified activated carbon (containing 12% as copper oxide) and 25% particulate activated carbon. The matrix formed is then treated with up to 19% w/w acrylonitrile binder. The filtration layer 2 is sandwiched between two layers, 3 and 4, of a net-like thermofusible web composed of ethyl vinyl acetate. Laminated on the outer surface of each of the thermofusible layers 3 and 4 is a 75 micron co-extruded film, 5 and 6, composed of ethyl vinyl acetate/polyvinyl dichloride/ ethyl vinyl acetate.

The filter 1 which is in the form of a disc having a diameter of 25mm is positioned on the inside of one wall 7 of an ostomy bag . The wall 7of the ostomy bag is composed of a film of ethyl vinyl acetate/polyvinyl dichloride/ ethyl vinyl acetate. In order to attach the filter 1 to the wall 7, heat is applied using a welding tool. Combined with the welding tool is a knife-edge that is positioned centrally so as to punch a hole through the bag wall 7 and through one of the co-extruded film layers 6. Thus gas can flow into the filtration layer 2 of the filter 1 around the perimeter and out of the centre of the filter 1 via a hole 8.

### Example 1

### Testing of Deodorising Capability of Filters

#### Purpose of evaluation:

Principally, to assess the chemisorption and physisorption properties of the filter materials supplied for the study.

Also, to compare the filters with a standard sample from "Charcoal Cloth" and with a Dansac sample.

**Samples**

Filters "40" 0% chemically modified carbon : 100% unmodified carbon
Filters "41" 25% chemically modified carbon : 75% unmodified carbon
Filters "42" 50% chemically modified carbon : 50% unmodified carbon
Filters "43" 75% chemically modified carbon : 25% unmodified carbon
Filters "44" 100% chemically modified carbon : 0% unmodified carbon
Charcoal Cloth "01"
Dansac Pouches

(The Charcoal Cloth International Ltd filters and The Dansac Pouch filter comprise 100% activated carbon impregnated with salts reactive to hydrogen sulphide but differ in construction.)

All of the filter samples were supplied as 25mm discs welded to a sheet of film. Into one side was punched a 3mm slit to enable the gas to pass through the filters. The filters were designed such that gas flowed in through the exposed sides of the filter and out through the central slit.

The filters on the Dansac pouch were squares of 20mm x 20mm, and were cut out of the pouch to be tested in the same manner as the other filters. The gas exit path on the Dansac filters was noted to be circular with a diameter of between 4mm-5mm.

**Test Methods:**

**Physisorption Testing**

Physisorption capacity testing at 500ml/min. GC grade Nitrogen containing $288 \pm 32$ppm of menthol.

The filter is deemed to have failed when 32ppm menthol passes through the filter (equivalent to detecting 50ng per 50$\mu$l sample injection).

See Appendix for more information relating to the physisorption testing of the filters.

**Chemisorption Testing**

$H_2S$ deodorising capability at 250ml/min flow rate. A mixture of nitrogen (80%) and methane (20%) with $25 \pm 2$ppm $H_2S$.

Filters which are designed to use a radial gas flow path (as in this case) are normally tested at 500ml/minute in order to reduce the length of time required to test the filters. However, these samples were all tested at 250ml/min. The test ends and the filter is deemed to have failed when 2ppm of $H_2S$ passes through the filter.

**Results:**

**Physisorption test results:** Minutes to reach 2ng/50$\mu$l sample:

*Table 1*

| Sample Type | Sample 1 | Sample 2 | Sample 3 |
| --- | --- | --- | --- |
| 40 | 107 | 116 | 122 |
| 41 | 102 | 100 | - |
| 42 | 82 | 82 | 86 |
| 43 | 82 | 68 | 64 |
| 44 | 80 | 64 | 90 |
| 01 | 14 | 14 | - |
| Dansac | 70 | 89 | 84 |

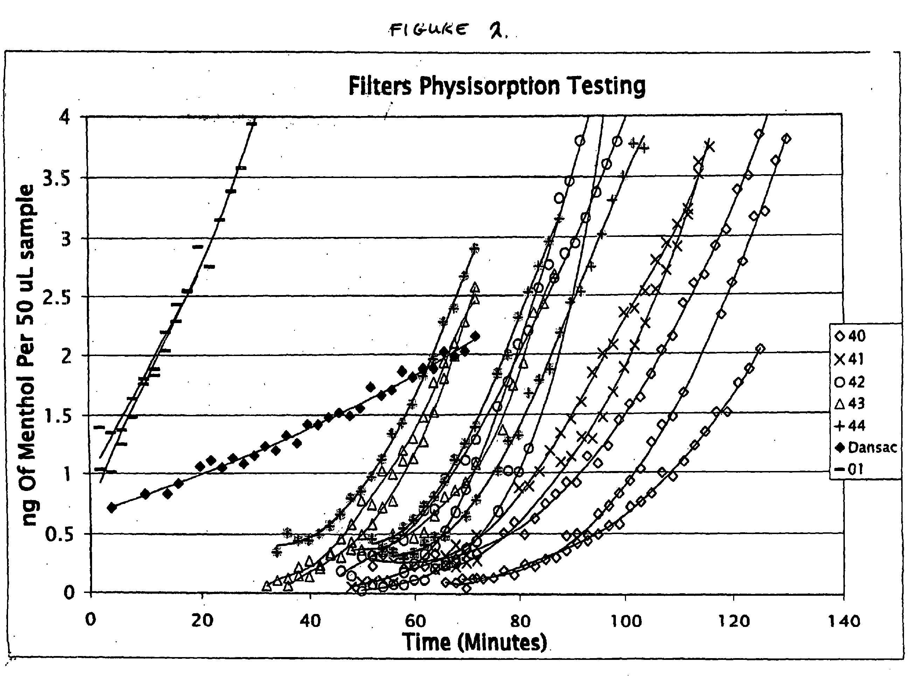

The raw data was averaged and plotted onto a graph: see Figure 2.

From the graph in Figure 2 it can clearly be seen that the worst performing filter is the standard charcoal cloth sample, since it immediately allowed a high concentration of menthol through. The Dansac filter also allowed some menthol to pass and the level rose gradually. All of the test samples "40" to "44" were considered to have performed better than either "01" (the standard charcoal cloth) or the Dansac filter. Of all the samples tested, there was a clear trend in the performance:

$$40>41>42>43>44$$

It should be noted that, although 2ng/50µl sample is chosen as the failure point, many organic odours would most likely be detectable to the human nose at lower levels, hence the failure profile shown by the Dansac filters is considered to be much worse than that of (for example) "42" since the Dansac filter allowed some menthol to pass right from the beginning of the test. "42" allowed no odour to pass until beginning to fail sharply after 40 minutes.

## Chemisorption Test Results

Hydrogen sulphide testing: Minutes to 2ppm $H_2S$:

### Table 2

| Sample Type | Sample 1 | Sample 2 | Sample 3 |
|---|---|---|---|
| 40 | 3 | 1 | 3 |
| 41 | 24 | 18 | 21 |
| 42 | 24 | 27 | 20 |
| 43 | 66 | 54 | 56 |
| 44 | 104 | 83 | 104 |
| 01 | 27 | 22 | 8 |
| Dansac | 227 | 272 | 227 |

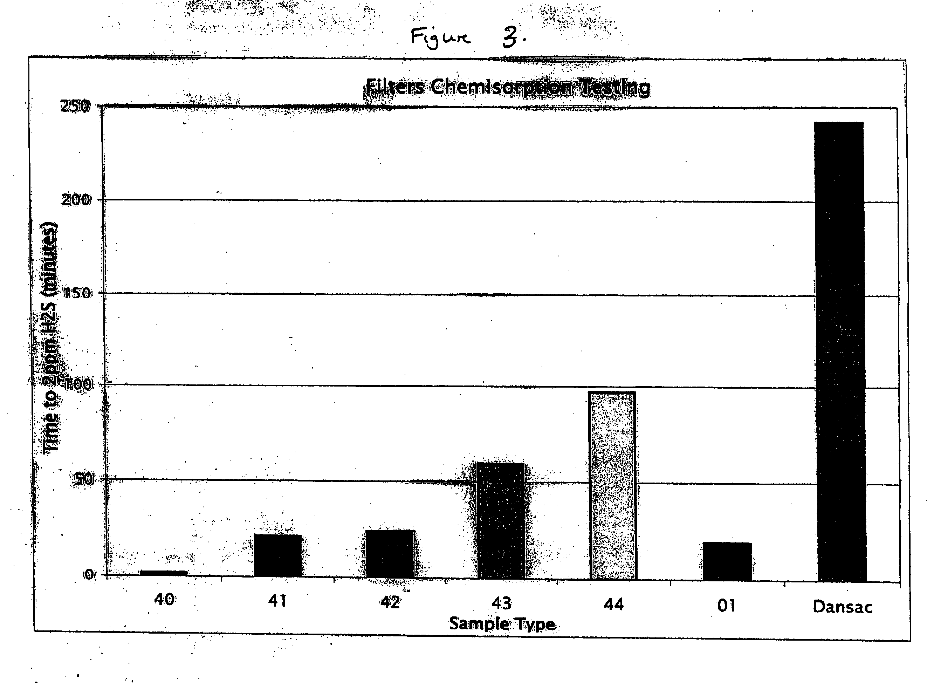

The average results were plotted onto a graph: see Figure 3.

The graph in Figure 3 clearly shows that the Dansac filter performed better than any other sample, and that the "01" filter again performed badly.

This, taken along with the physisorption results, suggests that the Dansac filter consists entirely of carbon activated to adsorb $H_2S$.

Interestingly, the "40" to "44" filters were ranked in the opposite order to the physisorption testing, thus:

$$44>43>42>41>40$$

this is not surprising considering the different loadings of the filters, and the results can be summarised as follows:

### Table 3

| Sample Type | Chemisorption Rank | Physisorption Rank |
|---|---|---|
| 40 | 5th | 1st |
| 41 | 4th | 2nd |
| 42 | 3rd | 3rd |
| 43 | 2nd | 4th |

(continued)

| Sample Type | Chemisorption Rank | Physisorption Rank |
|---|---|---|
| 44 | 1st | 5th |

**Conclusions:**

- It was possible to find a clear trend in the performance of the different types of filter in the physisorption and chemisorption tests.

- The standard Charcoal Cloth filter "01" was found to perform very poorly in every test.

- The Dansac filter out-performed all of the other filter types in the chemisorption test, but was poor in physisorption tests. It is probably composed only of carbon which has been activated specifically to adsorb $H_2S$.

**Appendix**

*Justification for the use of menthol as a suitable physisorption test material:*

Ideally, the physisorption test should consist of materials which the filter will be required to remove in real-life use. Such substances would for example be indole, skatole or other organic compounds produced by the human digestive system.

So far, it has not been possible to devise a suitable test method which uses such materials due to problems with low vapour pressures (which mean that each filter test run can last for many hours) and equipment contamination (which makes accurate run-run reproducibility impossible).

It was therefore decided to use a substance similar in molecular weight and structure to some of these compounds but which was more suited to the test procedure. It is also important that the substance be removed from the gas stream by physisorption alone, i.e. it does not interact with the chemisorption-activated carbon. Menthol satisfies these criteria:

*Table 5*

| Material | Molecular wt |
|---|---|
| Menthol | 156 |
| Indole | 117 |
| Skatole | 131 |

- All these materials are based around a reasonably rigid 6-carbon skeleton, and can be expected to have similar affinities for activated carbon.

- The extra ingredients in the carbon are specific to $H_2S$ removal, for example:

$$CuO+H_2S \rightarrow CuS+H_2O$$

Depending on the active substance, water is often released during the reaction with $H_2S$. Menthol does not react in this way.

- It is also apparent from the results that $H_2S$ and menthol are removed by different mechanisms. $H_2S$-removing carbon will generally adsorb *some* menthol since the gas will still be attracted to the carbon (e.g. sample "44", above, with 100% $H_2S$ activated with for example: metal compounds (e.g. "40", with no $H_2S$-activated carbon).

### Example 2

#### Patient Trial of Filter

Odour and security of a stoma appliance are two of the most major concerns for a patient following formation of a colostomy. Research from as far back as 1985 states these two concerns to be ranked in the top three concerns for a patient.

The objective of the trial was to show that the new filter in accordance with the invention was more effective in dealing with the two very different types of odour that faeces produce when compared to the users usual pouch.

#### Trialists

A simple tick box questionnaire was sent to thirty 'ostomists'. To date, twenty have responded.

All colostomists were given five of each type of pouch to use.

Other appliances used included Convatec, Dansac, Braun and Coloplast.

All trialists have all had their colostomies for varying periods of time, i.e. one to eight years and were competent in the management of their stomas.

Two different filters were tested. The new filter is designed to deal with the two very different types of odour that faeces produces. These being in origin, cyclic organics that are dealt with by pores in the carbon of the filter and the sulphurous chemicals eliminated by the chemically activated part of the filter.

The trial involved ostomists trying two different filters. The filters had varying ratios of chemically modified and unmodified activated carbon within them.

Filter "A" consisted of a 50/50 blend of modified and unmodified carbon, whilst Filter "B" consisted of 75/25 blend of modified and unmodified carbon.

#### RESULTS OF THE QUESTIONNAIRE

#### The Filter

When Trialling the New Filter

19/20 90% people found the filter on pouch B better or equivalent to that of their usual pouch;

15/20 trialists found filter B better; and

5/20 trialists found filter B equivalent to their usual pouch.

#### Claims

1.  An odour absorbing filter for use in medical appliances comprising a filtration layer formed of a matrix or substrate impregnated with activated carbon together with a binder and sandwiched between cover layers **characterized in that** the matrix is impregnated with a mixture comprising from 50 to 95% w/w particulate chemically modified activated carbon and from 5 to 50% w/w particulate activated carbon so that the ratio of chemically modified activated carbon to activated carbon is from 50:50 to 95:5.

2.  The filter according to Claim 1, wherein the matrix is impregnated with a mixture comprising from 70 to 87% w/w particulate chemically modified activated carbon and from 13 to 30% w/w particulate activated carbon so that the ratio of chemically modified activated carbon to activated carbon is from 70:30 to 87:13.

3.  The filter according to Claim 2, wherein the matrix is impregnated with a mixture comprising from 70 to 80% w/w particulate chemically modified activated carbon and from 20 to 30% w/w particulate activated carbon so that the ratio of chemically modified activated carbon to activated carbon is from 70:30 to 80:20.

4.  The filter according to any one of Claim 1 to 3, wherein the matrix is impregnated with a mixture comprising 75% w/w particulate chemically modified activated carbon and 25% w/w particulate activated carbon.

5.  The filter according to any one of Claims 1 to 4, wherein the matrix or substrate is selected from non-woven, woven or knitted fabric or a foam.

6. The filter according to any one of the preceding claims, wherein the chemically modified activated carbon is obtainable by impregnating a starting material with copper nitrate.

7. The filter according to Claim 6, wherein the starting material is impregnated with from 9 to 15% w/w measured as copper oxide using atomic absorption spectroscopy.

8. An ostomy bag incorporating an odour absorbing filter according to any one of Claims 1 to 7.

9. A wound dressing incorporating an odour absorbing filter according to any one of Claims 1 to 7.


**Patentansprüche**

1. Geruchsabsorbierender Filter zur Verwendung in medizinischen Geräten, der eine Filtrationsschicht, die aus einer Matrix oder einem Substrat gebildet ist, die bzw. das mit Aktivkohle imprägniert ist, zusammen mit einem Bindemittel umfasst und die zwischen Deckschichten positioniert ist, **dadurch gekennzeichnet, dass** die Matrix mit einer Mischung imprägniert ist, die aus 50 bis 95 Gew.-% Dispersionsteilchen, die chemisch modifizierter Aktivkohle sind und aus 5 bis 50 Gew.-% Dispersionsteilchen Aktivkohle besteht, sodass das Verhältnis chemisch modifizierter Aktivkohle zu Aktivkohle von 50:50 bis 95:5 ist.

2. Filter nach Anspruch 1, wobei die Matrix mit einer Mischung imprägniert ist, die von 70 bis 87 Gew.-% Dispersionsteilchen, die chemisch modifizierte Aktivkohle sind und von 13 bis 30 Gew.-% Dispersionsteilchen Aktivkohle umfasst, sodass das Verhältnis chemisch modifizierter Aktivkohle zu Aktivkohle von 70:30 bis 87:13 ist.

3. Filter nach Anspruch 2, wobei die Matrix mit einer Mischung imprägniert ist, die von 70 bis 80 Gew.-% Dispersionsteilchen, die chemisch modifizierte Aktivkohle sind und von 20 bis 30 Gew.-% Dispersionsteilchen Aktivkohle umfasst, sodass das Verhältnis chemisch modifizierter Aktivkohle zu Aktivkohle von 70:30 bis 80:20 ist.

4. Filter nach einem der Ansprüche 1 bis 3, wobei die Matrix mit einer Mischung imprägniert ist, die 75 Gew.-% Dispersionsteilchen chemisch modifizierter Aktivkohle und 25 Gew.-% Dispersionsteilchen Aktivkohle umfasst.

5. Filter nach einem der Ansprüche 1 bis 4, wobei die Matrix oder das Substrat aus Vliesstoff, Gewebe oder Gestrick oder einem Schaumstoff ausgewählt ist.

6. Filter nach einem der vorhergehenden Ansprüche, wobei die chemisch modifizierte Aktivkohle durch Imprägnieren eines Ausgangsmaterials mit Kupfernitrat erhältlich ist.

7. Filter nach Anspruch 6, wobei das Ausgangsmaterial mit von 9 bis 15 Gew.-%, gemessen als Kupferoxid unter Verwendung von Atomabsorptionsspektroskopie, imprägniert ist.

8. Ostomy-Beutel mit einem geruchsabsorbierenden Filter nach einem der Ansprüche 1 bis 7.

9. Wundverband mit einem geruchsabsorbierenden Filter nach einem der Ansprüche 1 bis 7.


**Revendications**

1. Filtre absorbant les odeurs prévu pour être utilisé dans des appareillages médicaux comprenant une couche de filtration formée d'une matrice ou d'un substrat imprégné de charbon actif avec un liant et pris en sandwich entre des couches de fermeture **caractérisé en ce que** la matrice est imprégnée d'un mélange comprenant 50 à 95% p/p de charbon actif particulaire modifié chimiquement et de 5 à 50% p/p de charbon actif particulaire de manière à ce que le rapport charbon actif modifié chimiquement à charbon actif soit de 50:50 à 95:5.

2. Filtre selon la Revendication 1, où la matrice est imprégnée d'un mélange comprenant 70 à 87% p/p de charbon actif particulaire modifié chimiquement et de 13 à 30% p/p de charbon actif particulaire de manière à ce que le rapport charbon actif modifié chimiquement à charbon actif soit de 70:30 à 87:13.

3. Filtre selon la revendication 2, où la matrice est imprégnée d'un mélange comprenant 70 à 80% p/p de charbon actif particulaire modifié chimiquement et de 20 à 30% p/p de charbon actif particulaire de manière à ce que le rapport charbon actif modifié chimiquement à charbon actif soit de 70:30 à 80:20.

4. Filtre selon l'une quelconque des Revendications 1 à 3, où la matrice est imprégnée d'un mélange comprenant 75% de charbon actif particulaire modifié chimiquement et 25% p/p de charbon actif particulaire.

5. Filtre selon l'une quelconque des revendications 1 à 4, où la matrice ou le substrat est sélectionné à partir d'un tissu tricoté, tissé ou non tissé ou d'une mousse.

6. Filtre selon l'une quelconque des revendications précédentes, où le charbon actif modifié chimiquement peut être obtenu en imprégnant un matériau de départ de nitrate de cuivre.

7. Filtre selon la Revendication 6, où le matériau de départ est imprégné de 9 à 15% p/p d'oxyde de cuivre en utilisant la spectroscopie d'absorption atomique.

8. Sac de stomie incorporant un filtre absorbant les odeurs selon l'une quelconque des Revendications 1 à 7.

9. Pansement de plaies incorporant un filtre absorbant les odeurs selon l'une quelconque des Revendications 1 à 7.

FIGURE I.,

FIGURE 2.

Filters Physisorption Testing

Legend:
◇ 40
✕ 41
○ 42
△ 43
+ 44
◆ Dansac
— 01

Y-axis: ng Of Menthol Per 50 uL sample
X-axis: Time (Minutes)

EP 1 380 333 B1

Figure 3.

**Filters Chemisorption Testing**

Y-axis: Time to 2ppm H2S (minutes), 0, 50, 100, 150, 200, 250

X-axis (Sample Type): 40, 41, 42, 43, 44, 01, Dansac

**EP 1 380 333 B1**

## REFERENCES CITED IN THE DESCRIPTION

### Patent documents cited in the description

- EP 0191646 A
- US 5281437 A